(19) Europäisches Patentamt
European Patent Office
Office européen des brevets

(11) Veröffentlichungsnummer: **0 142 739**
**B1**

(12) **EUROPÄISCHE PATENTSCHRIFT**

(45) Veröffentlichungstag der Patentschrift: **07.01.88**

(51) Int. Cl.⁴: **C 07 D 209/42**, C 07 D 215/48, C 07 D 401/12, A 61 K 31/40, A 61 K 31/47

(21) Anmeldenummer: **84112848.1**

(22) Anmeldetag: **13.09.80**

(60) Veröffentlichungsnummer der früheren Anmeldung nach Art. 76 EPÜ: **0029488**

(54) **Aminosäurederivate und Verfahren zu ihrer Herstellung.**

(30) Priorität: **19.09.79 DE 2937779**
**21.11.79 DE 2946909**

(43) Veröffentlichungstag der Anmeldung: **29.05.85 Patentblatt 85/22**

(45) Bekanntmachung des Hinweises auf die Patenterteilung: **07.01.88 Patentblatt 88/1**

(84) Benannte Vertragsstaaten: **AT BE CH DE FR GB IT LI NL SE**

(56) Entgegenhaltungen:
**DE - A - 2 703 828**
**FR - A - 2 448 533**

**Die Akte enthält technische Angaben, die nach dem Eingang der Anmeldung eingereicht wurden und die nicht in dieser Patentschrift enthalten sind.**

(73) Patentinhaber: **HOECHST AKTIENGESELLSCHAFT, Postfach 80 03 20, D-6230 Frankfurt am Main 80 (DE)**

(72) Erfinder: **Geiger, Rolf, Prof. Dr., Heinrich-Bleicher-Strasse 33, D-6000 Frankfurt am Main 50 (DE)**
Erfinder: **Teetz, Volker, Dr., An der Tann 20, D-6238 Hofheim am Taunus (DE)**
Erfinder: **Schölkens, Bernward, Dr., Am Fliedergarten 1, D-6233 Kelkheim (Taunus) (DE)**
Erfinder: **Wissmann, Hans, Dr., Falkenstrasse 12, D-6232 Bad Soden am Taunus (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

**Beschreibung**

Die Erfindung betrifft Verbindungen der Formel I

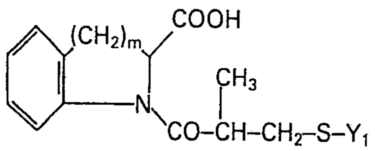

(I)

in welcher m = 1 oder 2 ist und $Y_1$ = Acetyl, Nicotinoyl oder, falls m = 2 ist, ausserdem Wasserstoff, Benzoyl oder einen Rest der Formel II

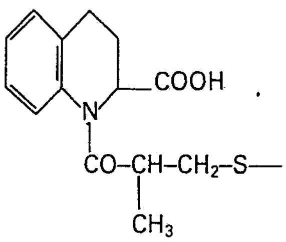

(II)

bedeutet sowie deren Salze.

Die Verbindungen besitzen ähnlich wie viele andere Derivate des Indolins und des 1,2,3,4-Tetrahydrocholins wertvolle pharmakologische Eigenschaften.

Die Erfindung betrifft ferner ein Verfahren zur Herstellung dieser Verbindungen. Dieses ist dadurch gekennzeichnet, dass man einen ($C_1$–$C_4$)-Alkylester einer Verbindung der Formel III

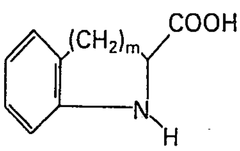

(III)

oder ein Salz dieser Verbindung mit einer starken nicht flüchtigen organischen Base mit einem aktivierten Derivat einer Carbonsäure der Formel IV

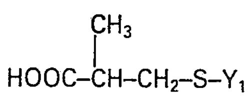

(IV)

in der $Y_1 \neq H$ ist, umsetzt, aus dem erhaltenen Produkt die organische Base bzw. die Estergruppe abspaltet, gegebenenfalls einen in der Position $Y_1$ stehenden Säurerest abspaltet oder durch einen anderen austauscht und, falls die Verbindung eine freie SH-Gruppe hat, gewünschtenfalls das Produkt zur Disulfidverbindung oxidiert und die Verbindung der Formel I gegebenenfalls in ihre Salze überführt.

Die Synthese der erfindungsgemässen Verbindungen ist einfach. Die als Ausgangsprodukt in Frage kommende Indolin-2-carbonsäure ist aus Aust. J. Chem. 20 (1967), Seite 1935, die 1,2,3,4-Tetrahydrochinolin-2-carbonsäure aus Ber. 61 (1928), Seite 2377 bekannt. Die Thiolan-carbonsäure der Formel IV kennt man aus Acta Chem. Scand. 9 (1955), Seite 178 und 10 (1956), Seite 678.

Die Carbonsäure der Formel IV sind unter anderem in der DE-OS 2752720 beschrieben oder können analog hergestellt werden.

Die Herstellung der Säureamidbindung folgt den allgemeinen Verfahren, bevorzugt denen der Peptidchemie, wie sie in Houben-Weyl, Methoden der Organischen Chemie, Band 15, ausführlich beschrieben sind.

Als aktivierte Derivate einer Carbonsäure der Formel IV kommen z.B. die Säurechloride, Aktivester, gemischte Anhydride mit beliebigen anderen Carbonsäuren oder Kohlensäurehalbester in Frage. Sie lassen sich mit Salzen der Aminosäuren der Formel III, speziell dem tert.-Butylester oder mit Basensalzen, z.B. Tetralkylguanidinium-, Trialkylbenzyl- oder Tetraalkylammoniumsalzen direkt umsetzen.

Aktivierte Ester werden z.B. aus der Säure und N-Hydroxysuccinimid, 2,4,5-Trichlorbenzol oder 1-Hydroxybenzotriazol in bekannter Weise mit Dicyclohexylcarbodiimid hergestellt und nach dem Abfiltrieren des bei der Herstellung entstehenden Dicyclohexylharnstoffes in Lösung sogleich verwendet. Bevorzugte Lösungsmittel sind Dimethylformamid, Dimethylacetamid, N-Methylpyrrolidon, Phosphorsäure-tris-dimethylamid oder Methylenchlorid. In denselben Lösungsmitteln werden die erfindungsgemässen Salze der Aminosäuren der Formel III aufgenommen, die zuvor z.B. in alkoholischer oder wässrig-alkoholischer Lösung aus den Komponenten in äquimolarer Menge erhalten und vom Lösungsmittel durch Abdestillieren im Vakuum befreit wurden.

Starke, nicht flüchtige organische Basen im Sinne der Erfindung sind insbesondere Tetraalkyl- oder aralkylammoniumhydroxide oder tetraalkylierte Guanidine wie Tetramethylguanidin.

Verbindungen, in denen $Y_1$ Wasserstoff ist, können durch Behandeln der entsprechenden S-Acylderivate mit Ammoniak oder Alkali- bzw. Erdalkalihydroxiden erhalten werden. Mit den letzten Agentien werden auch gleichzeitig evtl. vorhandene Methyl- oder Ethylester-Gruppen verseift.

Die Reaktion kann bei Raumtemperatur stattfinden und ist bei Verwendung der Säurechloride nach etwa 30 Minuten, bei Verwendung von 1-Hydroxybenzotriazolestern nach längstens 4 Stunden beendet. Man destilliert dann das Lösungsmittel im Vakuum ab und reinigt das Rohprodukt z.B. durch Flüssigkeitschromatographie an Silicagel, wobei sich Lösungsmittelsysteme mit Chloroform, Methanol und Essigsäure bewährten.

Man kann aber auch, wie das bevorzugt in der Peptidchemie geschieht, die Carbonsäuren IV mit Alkylestern der Aminosäuren der Formel III kondensieren und die Ester anschliessend spalten oder verseifen, wobei im allgemeinen gleichzeitig die S-Acylbindung gespalten wird. Bevorzugtes Kondensationsmittel ist Dicyclohexylcarbodiimid, gegebenenfalls unter Zusatz von N-Hydroxysuccinimid oder 1-Hydroxybenzotriazol. Bevorzugte Lösungsmittel sind die oben aufgeführten.

Die erhaltenen Produkte können gegebenenfalls weiter modifiziert werden durch Abspaltung von Acylgruppen wie Acetyl oder Benzoyl in der Position $Y_1$, z.B. durch Behandeln mit Ammoniak, einem Alkali- oder Erdalkalihydroxid und gegebenenfalls Behandeln mit einem milden Oxidationsmittel wie Luft, Jod oder Kaliumhexacyanoferrat-(III) zwecks Einführung eines Restes der Formel II.

Verbindungen der Formel I, in denen $Y_1$ Nicotinoyl darstellt, können auch erhalten werden, indem man eine entsprechende Verbindung der Formel III mit einem aktivierten Derivat der Nicotinsäure umsetzt.

Je nach dem Charakter der Ausgangsstoffe, insbesondere der Bedeutung des Substituenten $Y_1$, kann das eine oder andere der beschriebenen Verfahren in einzelnen Fällen eine gewünschte individuelle Verbindung nur in geringen Ausbeuten liefern oder zu deren Synthesen nicht geeignet sein. In solchen verhältnismässig selten auftretenden Fällen macht es dem Fachmann keine Schwierigkeiten, das gewünschte Produkt auf einem anderen der beschriebenen Verfahrenswege zu synthetisieren.

Die erfindungsgemässen Verbindungen besitzen 1–2 chirale Zentren. Sie liegen zunächst als Harze vor und lassen sich zum Teil durch längeres Behandeln mit Petrolether in feste Form überführen. Sie schmelzen meist unter Zersetzung und besitzen einen unscharfen Schmelzpunkt, der zudem stark von der Heizdauer abhängt. Ihre Identität wird durch Elementaranalyse, UV-, IR- und NMR-Spektroskopie gesichert.

Durch Gegenstromverteilung ihrer Salze mit optisch aktiven Basen konnten stereochemisch einheitlichere Verbindungen mit höherer biologischer Aktivität erhalten werden, deren absolute Konfiguration noch unbekannt ist. In praxi kann die Anwendung der stark und besonders langwirksamen Verbindungen auch in Form des Stereoisomerengemisches erfolgen.

Die erfindungsgemässen Verbindungen erniedrigen bei peroraler Gabe von 1 bis 20 mg pro kg und Tag an der hypertonen Ratte und am Hund den Blutdruck und sind auch am Menschen in demselben Dosierbereich wirksam. Die Dosis konnte im Tierversuch ohne schädliche Wirkung erhöht werden.

Die neuen Verbindungen werden zur Bekämpfung des Bluthochdrucks verschiedener Genese eingesetzt. Sie können für sich oder in Kombination mit anderen blutdrucksenkenden, gefässerweiternden oder diuretisch wirksamen Verbindungen angewandt werden. Typische Vertreter dieser Wirkklasse sind z.B. in Ehrhardt-Ruschig, Arzneimittel, 2. Auflage, Weinheim 1972, beschrieben.

Die Erfindung wird durch folgende Beispiele weiter erläutert:

Beispiel 1
3-Mercapto-2-methylpropanoyl-1,2,3,4-tetrahydrochinolin-2-carbonsäure

1,63 g 2-Methyl-3-acetylmercapto-propionsäure und 2,3 g 1,2,3,4-Tetrahydrochinolin-2-carbonsäuremethylesterhydrochlorid, hergestellt nach Ber. 61 (1928), Seite 2377, werden unter Stickstoff in 20 ml Dimethylacetamid gelöst. Man gibt ferner nacheinander 1,35 g 1-Hydroxy-benzotriazol, 1,28 ml N-Ethylmorpholin und 2,2 g Dicyclohexylcarbodiimid zu und rührt 4 Stunden bei Raumtemperatur. Dann filtriert man und destilliert aus dem Filtrat das Lösungsmittel im Vakuum ab. Der Rückstand wird in 30 ml Methanol gelöst. Man rührt und tropft unter Stickstoff 10 ml 4N NaOH zu und versetzt 10 Minuten nach beendeter Zugabe mit 10 ml 4N HCl. Dann wird das Gemisch im Vakuum zur Trockene gebracht. Nach Abdestillieren des Lösungsmittels reinigt man durch Chromatographie an 130 g $SiO_2$ im System Chloroform/Isopropanol/Essigsäure (50:10:3). Ausbeute 2,3 g. Elementaranalyse und NMR-Spektrum korrekt.

Beispiel 2
2-Methyl-3-nicotinoylmercaptopropionyl-1,2,3,4-tetrahydrochinolin-2-carbonsäure

Man löst 1,4 g der nach Beispiel 1 erhaltenen Verbindung in 10 ml Dimethylformamid und lässt unter Stickstoff mit 1,1 g Nicotinsäure-N-hydroxysuccinimidester (hergestellt in üblicher Weise aus Nicotinsäure, N-Hydroxysuccinimid und Dicyclohexyl-carbodiimid, Schmelzpunkt 137–138°C) reagieren. Nach 3 Stunden wird das Lösungsmittel abdestilliert und der Rückstand analog Beispiel 1 chromatographiert. Ausbeute 0,7 g. UV- und NMR-Spektrum weisen das Produkt als die gesuchte Verbindung aus.

Beispiel 3
2-Methyl-3-acetylmercaptopropionyl-indolin-2-carbonsäure

Man stellt aus 2-Methyl-3-acetylmercaptopropionsäure mit 10% Überschuss Thionylchlorid in Methylenchlorid durch dreistündiges Erwärmen das Säurechlorid her, das durch Destillation bei 1 mm Hg rein erhalten wird.

1,9 g des Säurechlorids werden mit dem Tetramethylenguanidiniumsalz von 1,63 g Indolin-2-carbonsäure in Dimethylacetamid unter Stickstoff umgesetzt. Man erhält nach Aufarbeitung und Reinigung analog Beispiel 1 2,9 g reine Titelverbindung mit korrekter Elementaranalyse.

**Patentansprüche für die Vertragsstaaten BE CH DE FR GB IT LI NL SE**

1. Verbindungen der Formel I

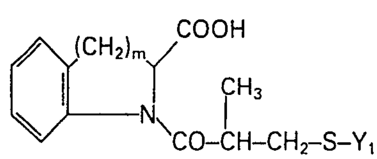 (I)

in welcher
m = 1 oder 2 ist und
$Y_1$ = Acetyl, Nicotinoyl oder, falls m=2 ist, ausserdem Wasserstoff, Benzoyl oder einen Rest der Formel II

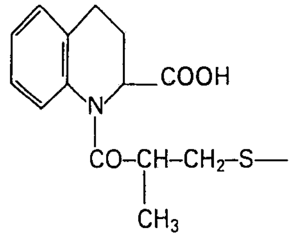 (II)

bedeutet
sowie deren Salze.

2. Verbindung gemäss Anspruch 1, in der m = 1 ist.

3. Verbindung gemäss Anspruch 1, in der m = 2 ist.

4. 3-Mercapto-2-methylpropanoyl-1,2,3,4-tetrahydrochinolin-2-carbonsäure und deren Salze.

5. 2-Methyl-3-nicotinoylmercaptopropionyl-1,2,3,4-tetrahydrochinolin-2-carbonsäure und deren Salze.

6. 3-Acetylmercaptopropionyl-2-methyl-indolin-2-carbonsäure und deren Salze.

7. Verfahren zur Herstellung einer Verbindung der Formel I gemäss Anspruch 1, dadurch gekennzeichnet, dass man einen ($C_1$-$C_4$)-Alkylester einer Verbindung der Formel III

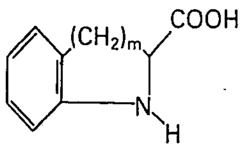
(III)

oder ein Salz dieser Verbindung mit einer starken, nicht flüchtigen organischen Base mit einem aktivierten Derivat einer Carbonsäure der Formel IV

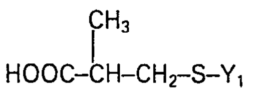
(IV)

in der $Y_1 \neq H$ ist, umsetzt, aus dem erhaltenen Produkt die organische Base bzw. die Estergruppe abspaltet, gegebenenfalls einen in der Position $Y_1$ stehenden Säurerest abspaltet oder durch einen anderen austauscht und, falls die Verbindung eine freie SH-Gruppe hat, gewünschtenfalls das Produkt zur Disulfidverbindüng oxidiert und die Verbindung der Formel I gegebenenfalls in ihre Salze überführt.

8. Verfahren gemäss Anspruch 7, dadurch gekennzeichnet, dass die organische Base mittels einer Säure oder eines stark sauren Ionenaustauschers abgespalten wird.

9. Verfahren gemäss Anspruch 7, dadurch gekennzeichnet, dass der in der Position $Y_1$ stehende Säurerest mit Alkali abgespalten wird.

10. Verbindung gemäss einem der Ansprüche 1–6 zur Anwendung als Heilmittel.

11. Verbindung gemäss einem der Ansprüche 1–6 zur Anwendung als Heilmittel bei der Behandlung des Bluthochdrucks.

12. Zubereitung enthaltend eine Verbindung gemäss einem der Ansprüche 1-6.

13. Zubereitung gemäss Anspruch 12 zur Anwendung als Heilmittel bei der Behandlung des Bluthochdrucks.

**Patentansprüche für den Vertragsstaat AT**

1. Verfahren zur Herstellung einer Verbindung der Formel I

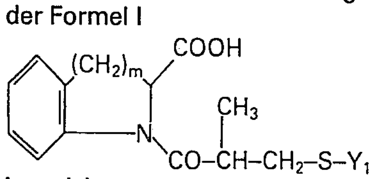
(I)

in welcher

m = 1 oder 2 ist und

$Y_1$ = Acetyl, Nicotinoyl oder, falls m=2 ist, ausserdem Wasserstoff, Benzoyl oder einen Rest der Formel II

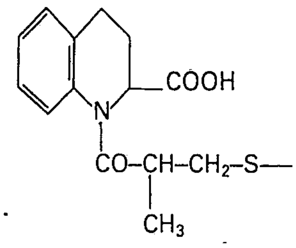
(II)

bedeutet

sowie deren Salze, dadurch gekennzeichnet, dass man einen ($C_1$-$C_4$)-Alkylester einer Verbindung der Formel III

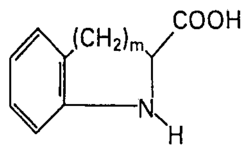
(III)

oder ein Salz dieser Verbindung mit einer starken, nicht flüchtigen organischen Base mit einem aktivierten Derivat einer Carbonsäure der Formel IV

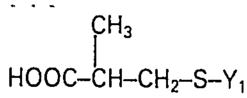
(IV)

in der $Y_1 \neq H$ ist, umsetzt, aus dem erhaltenen Produkt die organische Base bzw. die Estergruppe abspaltet, gegebenenfalls einen in der Position $Y_1$ stehenden Säurerest abspaltet oder durch einen anderen austauscht und, falls die Verbindung eine freie SH-Gruppe hat, gewünschtenfalls das Produkt zur Disulfidverbindung oxidiert und die Verbindung der Formel I gegebenenfalls in ihre Salze überführt.

2. Verfahren gemäss Anspruch 1, dadurch gekennzeichnet, dass eine Verbindung der Formel 1 hergestellt wird, in der m = 1 ist.

3. Verfahren gemäss Anspruch 1, dadurch gekennzeichnet, dass eine Verbindung der Formel 1 hergestell wird, in der m = 2 ist.

4. Verfahren gemäss Anspruch 1 oder 3, dadurch gekennzeichnet, dass 3-Mercapto-2-methylpropanoyl-1,2,3,4-tetrahydrochinolin-2-carbonsäure oder deren Salz hergestellt wird.

5. Verfahren gemäss Anspruch 1 oder 3, dadurch gekennzeichnet, dass 2-Methyl-3-nicotinoylmercaptopropionyl-1,2,3,4-tetrahydrochinolin-2-carbonsäure oder deren Salz hergestellt wird.

6. Verfahren gemäss Anspruch 1 oder 2, dadurch gekennzeichnet, dass 3-Acetylmercaptopropionyl-2-methylindolin-2-carbonsäure oder deren Salz hergestellt wird.

7. Verfahren gemäss einem der Ansprüche 1–6, dadurch gekennzeichnet, dass die organische Base mittels einer Säure oder eines strark sauren Ionenaustauschers abgespalten wird.

8. Verfahren gemäss einem der Ansprüche 1–5, dadurch gekennzeichnet, dass der in der Position $Y_1$ stehende Säurerest mit Alkali abgespalten wird.

9. Verfahren zur Herstellung einer Zubereitung enthaltend eine Verbindung der Formel I, in der m und $Y_1$ gemäss einem der Ansprüche 1–6 definiert sind, dadurch gekennzeichnet, dass man die Verbindung der Formel I in eine geeignete Darreichungsform bringt.

**Claims for the Contracting States BE, CH, DE, FR, GB, IT, LI, NL, SE**

1. Compounds of the formula I

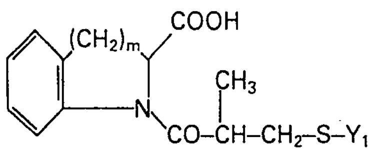

(I)

in which
m is 1 or 2
$Y_1$ denotes acetyl, nicotinoyl, or, if m is 2, also hydrogen, benzoyl or a radical of the formula II

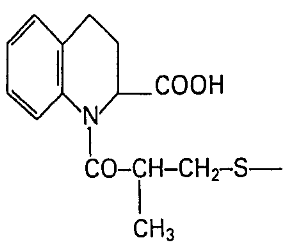

(II)

and their salts.

2. The compound according to in claim 1, in which m is 1.

3. The compound according to in claim 1, in which m is 2.

4. 3-Mercapto-2-methylpropanoyl-1,2,3,4-tetrahydroquinoline-2-carboxylic acid and its salts.

5. 2-Methyl-2-nicotinoylmercaptopropionyl-1,2,3,4-tetrahydroquinoline-2-carboxylic acid and its salts.

6. 2-Acetylmercaptopropionyl-2-methyl-indoline-2-carboxylic acid and its salts.

7. A process for the preparation of a compound of the formula as defined in claim 1, which comprises reacting ($C_1$-$C_4$)-alkyl ester of a compound of the formula III

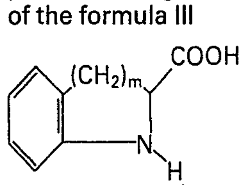

(III)

or a salt of such a compound with a strong non volatile organic base with an activated derivative of a carboxylic acid of the formula

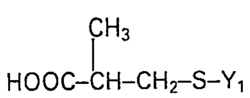

(IV)

in which $Y_1$ is not hydrogen, splitting off from the product obtained the organic base or the ester group, optionally splitting off an acid radical in position $Y_1$ or replacing same by another one and, if the compound carries a free SH group, oxidizing the compound, if appropriate, to give the disulfide compound, and optionally converting the compound of the formula I into its salts.

8. The process according to claim 7, characterized in that the organic base is split off by means of an acid or a strong acid ion exchanger.

9. The process according to claim 7, characterized in that the acid radical in position $Y_1$ is split off by means of alkali.

10. The compound according to any of claims 1–6 for the use as a medicament.

11. The compound according to any of claims 1–6 for the use as a medicament for treating high blood pressure.

12. A composition containing a compound according to any of claimx 1–6.

13. The composition according to claim 12 for the use as a medicament for treating high blood pressure.

**Claims for the Contracting state AT**

1. A process for the preparation of a compound of the formula I

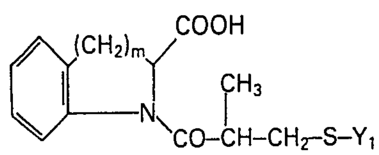

(I)

in which
m is 1 or 2
$Y_1$ denotes acetyl, nicotinoyl, or, if m is 2, also hydrogen, benzoyl or a radical of the formula II

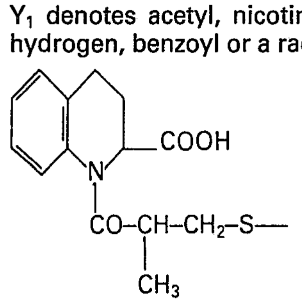

(II)

and their salts, which comprises reacting a ($C_1$-$C_4$)-alkyl ester of a compound of the formula III

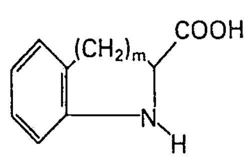

(III)

or a salt of such a compound with a strong non volatile organic base with an activated derivative of a carboxylic acid of the formula

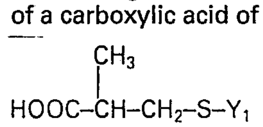

(IV)

in which $Y_1$ is not hydrogen, splitting off from the product obtained the organic base or the ester group, optionally splitting off an acid radical in position $Y_1$ or replacing same by another one and, if the compound carries a free SH group, oxidizing the compound, if appropriate, to give the disulfide compound, and optionally converting the compound of the formula I into its salts.

2. The process according to claim 1, characterized in that a compound of the formula I is prepared, in which m is 1.

3. The process according to claim 1, characterized in that a compound of the formula I is prepared in which m is 2.

4. The process according to claim 1 or 3, characterized in that 3-mercapto-2-methylpropanoyl-1,2,3,4-tetrahydroquinoline-2-carboxylic acid or its salt is prepared.

5. The process according to claim 1 or 3, characterized in that 2-methyl-3-nicotinoylmercaptopropionyl-1,2,3,4-tetrahydroquinoline-2-carboxylic acid or its salt is prepared.

6. The process according to claim 1 or 2, characterized in that 2-acetylmercaptopropionyl-2-methyl-indoline-2-carboxylic acid or its salt is prepared.

7. The process according to any of claims 1–6, characterized in that the organic base is split off by means of an acid or a strong acid ion exchanger.

8. The process according to any of claims 1–5, characterized in that the acid radical in position $Y_1$ is split off by means of alkali.

9. A process for the preparation of a composition containing a compound of the formula I, in which m and $Y_1$ are defined according to any of claims 1–6, characterized in that the compound of the formula I is converted in a suitable form for administration.

**Revendications pour les Etats contractants BE, CH, DE, FR, GB, IT, LI, NL, SE**

1. Composés de formule I

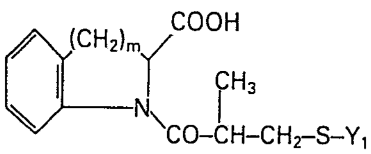

(I)

dans laquelle
m = 1 ou 2 et
$Y_1$ représente le groupe acétyle, nicotinoyle ou, au cas ou m = 2, en outre un atome d'hydrogène, le groupe benzoyle ou un radical de formule II

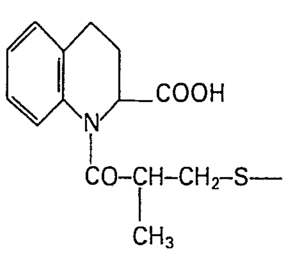

(II)

ainsi que leurs sels.

2. Composé selon la revendication 1, dans lequel m = 1.

3. Composé selon la revendication 1, dans lequel m = 2.

4. Acide 3-mercapto-2-méthylpropanoyl-1,2,3,4-tétrahydroquinoléine-2-carboxylique et ses sels.

5. 2-méthyl-3-nicotinoylmercaptopropionyl-1,2,3,4-tétrahydroquinoléine-2-carboxylic et ses sels.

6. Acide 3-acétylmercaptopropionyl-2-méthylindoline-2-carboxylique et ses sels.

7. Procédé pour la préparation d'un composé de formule I selon la revendication 1, caractérisé en ce que l'on fait réagir un ester alkylique (alkyle en $C_1$-$C_4$) d'un composé de formule III

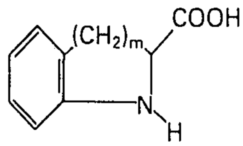

(III)

ou un sel de ce composé et d'une base organique forte non volatile, avec un dérivé actif d'un acide carboxylique de formule IV

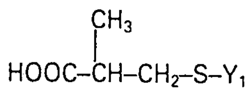

(IV)

dans laquelle $Y_1$ est différent de H, on élimine la base organique ou, respectivement, le groupe ester hors du produit obtenu, éventuellement on élimine un reste acide se trouvant en la position de $Y_1$, ou on le remplace par un autre, et au cas où le composé comporte un groupe SH libre, on oxyde si on le désire le produit en le composé disulfure et éventuellement on convertit le composé de formule I en ses sels.

8. Procéde selon la revendication 7, caractérisé en ce que l'on élimine la base organique au moyen d'un acide ou d'un échangeur d'ions fortement acide.

9. Procédé selon la revendication 7, caractérisé en ce que l'on sépare avec un alcali le reste acide se trouvant en le position de $Y_1$.

10. Composé selon l'une des revendications 1 à 6, à utiliser en tant que médicament.

11. Composé selon l'une des revendications 1 à 6, à utiliser en tant que médicament dans le traitement de l'hypertension.

12. Préparation contenant un composé selon l'une des revendications 1 à 6.

13. Préparation selon la revendication 12, à utiliser en tant que médicament dans le traitement de l'hypertension.

**Revendications pou l'Etat contractant At**

1. Procédé pour la préparation d'un composé de formule I

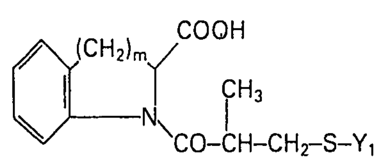

(I)

dans laquelle
m = 1 ou 2 et
$Y_1$ représente le groupe acétyle, nicotinoyle ou, au cas où m = 2, en outre un atome d'hydrogène, le groupe benzoyle ou un radical de formule II

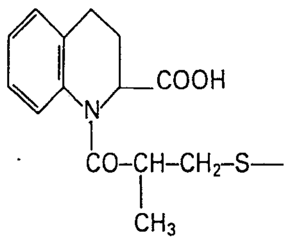

(II)

et des sels de celui-ci, caractérisé en ce que l'on fait réagir un ester alkylique (alkyle en $C_1$-$C_4$) d'un composé de formule III

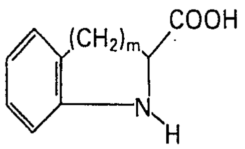

(III)

ou un sel de ce composé et d'une base organique forte non volatile, avec un dérivé actif d'un acide carboxylique de formule IV

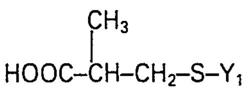

(IV)

dans laquelle $Y_1$ est différent de H, on élimine la base organique ou, respectivement, le groupe ester hors du produit obtenu, éventuellement on élimine un reste acide se trouvant en la position de $Y_1$, ou on le remplace par un autre, et au cas où le composé comporte un groupe SH libre, on oxyde si on le désire le produit en le composé disulfure et éventuellement on convertit le composé de formule I en ses sels.

2. Procédé selon la revendication 1, caractérisé en ce que l'on prépare un composé de formule I dans lequel m = 1.

3. Procédé selon la revendication 1, caractérisé en ce que l'on prépare un composé de formule I dans lequel m = 2.

4. Procédé selon la revendication 1 ou 3, caractérisé en ce que l'on prépare l'acide 3-mercapto-2-méthylpropanoyl-1,2,3,4-tétrahydroquinoléine-2-carboxylique ou un sel de celui-ci.

5. Procédé selon la revendication 1 ou 3, caractérisé en ce que l'on prépare l'acide 2-méthyl-3-nicotinoylmercaptopropionyl-1,2,3,4-tétrahydroquinoléine-2-carboxylique ou un sel de celui-ci.

6. Procédé selon la revendication 1 ou 2, caractérisé en ce que l'on prépare l'acide 3-acétylmercaptopropionyl-2-méthyl-indoline-2-carboxylique ou un sel de celui-ci.

7. Procédé selon l'une des revendications 1 à 6 caractérisé en ce que l'on élimine la base organique au moyen d'un acide ou d'un échangeur ions fortement acide.

8. Procédé selon l'une des revendications 1 à 5, caractérisé en ce que l'on sépare avec un alcali le reste acide se trouvant en la position de $Y_1$.

9. Procédé pour la préparation d'une composition contenant un composé de formule I, dans lequel m et $Y_1$ sont définis selon l'une des revendications 1 à 6, caractérisé en ce que l'on met le composé de formule I sous une forme d'administration appropriée.